Europäisches Patentamt

European Patent Office

Office européen des brevets

(11) Veröffentlichungsnummer: **0 375 091**
**A1**

## EUROPÄISCHE PATENTANMELDUNG

(21) Anmeldenummer: 89250116.4

(22) Anmeldetag: 18.12.89

(51) Int. Cl.5: **C12N 1/21, C12N 15/82, C12N 15/70, A01H 5/00, A01N 65/00**

Der Mikroorganismus ist bei DSM unter der Nummer 5088 hinterlegt worden.

(30) Priorität: 21.12.88 DE 3843628

(43) Veröffentlichungstag der Anmeldung: 27.06.90 Patentblatt 90/26

(84) Benannte Vertragsstaaten:
AT BE CH DE ES FR GB GR IT LI LU NL SE

(71) Anmelder: INSTITUT FÜR GENBIOLOGISCHE FORSCHUNG BERLIN GMBH
Ihnestr.63
D-1000 Berlin 33(DE)

(72) Erfinder: Keil, Michael, Dr.
Phys. and Biol. Research Div. Shell Research Ltd.
Sittingbourne Kent ME 9 8 A 6(GB)
Erfinder: Sanchez-Serrano, José, Dr.
Offenbacher Strasse 6
D-1000 Berlin 33(DE)
Erfinder: Willmitzer, Lothar, Prof.Dr.
Am Kleinen Wannsee 34
D-1000 Berlin 39(DE)

(54) Wundinduzierbare und kartoffelknollenspezifische transkriptionale Regulation.

(57) Es wird eine neue DNA-Sequenz einer Expressions-Kassette auf der die regulatorischen Regionen für die wundinduzierbare transkriptionale Regulation im Stengel und in Blättern sowie für die konstitutive transkriptionale Regulation in der Kartoffelknolle lokalisiert sind, sowie die Übertragung dieser in Vektoren enthaltenen DNA-Sequenz in das pflanzliche Genom, unter Verwendung von Agrobakterium tumefaciens als Transfer-Mikroorganismus, beschrieben. Die übertragene DNA-Sequenz sorgt sowohl für die Regulierung endogener Produkte als auch für die Herstellung heterologer Produkte. Die Methode findet besondere Verwendung in Verbindung mit der Expression schädlingsresistenter Gene in Blättern, Stengeln und Knollen nach einer Verwendung und für die Produktion nützlicher Proteine in Kartoffelknollen.

## Wundinduzierbare und kartoffelknollenspezifische transkriptionale Regulation

Die vorliegende Erfindung betrifft eine neue DNA-Sequenz einer Expressions-Kassette, auf der die regulatorischen Regionen für die wundinduzierbare transkriptionale Regulation im Stengel und in Blättern sowie für die konstitutive transkriptionale Regulation in der Kartoffelknolle lokalisiert sind, sowie die Übertragung dieser in Vektoren enthaltenen DNA-Sequenz in das pflanzliche Genom, unter Verwendung von Agrobakterien als Transfer-Mikroorganismen. Die übertragene DNA-Sequenz sorgt sowohl für die Regulierung endogener Produkte als auch für die Herstellung heterologer Produkte. Heterologe Produkte können z.B. toxische Proteine sein, die zur Abwehr von Pflanzenschädlingen verwendet werden können.

Bedingt durch den kontinuierlich steigenden Nahrungsmittel- und Rohstoffbedarf, der aus der ständig wachsenden Weltbevölkerung resultiert, wird es in zunehmendem Maße Aufgabe der biotechnologischen Forschung, sich wegen der langfristig abzusehenden sich verkleinernden Anbauflächen um die Steigerung des Ertrages von Nutzpflanzen und deren Inhaltsstoffe zu bemühen. Eine Steigerung des Ertrages kann u.a. dadurch erreicht werden, daß die Resistenz von Nutzpflanzen gegen Pflanzenschädlinge und Pflanzenkrankheiten und/oder ungünstige Bodenverhältnisse verstärkt wird. Eine Verstärkung der Resistenz könnte z.B. dadurch erreicht werden, daß die Pflanzen induziert und zu einer vermehrten Bildung von Schutzstoffen veranlaßt werden. Hierzu muß der Metabolismus der Pflanze manipuliert werden. Dies kann u.a. durch die Veränderung der im Zellkern enthaltenen Desoxyribonukleinsäure (DNA) hervorgerufen werden. Oft wäre es wünschenswert, in den DNA-Abschnitten, die für die Transkription in einem oder mehreren Teilbereichen der Pflanze oder während eines bestimmten Zeitabschnitts im Pflanzenwachstumszylkus verantwortlich sind, einzugreifen. Daher besteht ein großes Interesse an der Indentifizierung der für die Transkription oder Expression endogener Pflanzenprodukte verantwortlichen DNA-Sequenzen im Pflanzengenom. Um derartige DNA-Sequenzen herauszufinden, muß man zunächst nach. Produkten suchen, die zu einer bestimmten Zeit im Zellwachstumszyklus oder in einem bestimmten Teil der Pflanze erscheinen. Hat man die dazugehörenden Gene identifiziert und isoliert, ist eine gründliche Untersuchung der Sequenz und vor allem die Identifizierung und Isolierung der gewünschten transkriptionalen regulatorischen Regionen erforderlich. Anschließend müssen geeignete Modelle erstellt werden, deren Funktionen durch Versuche nachgewiesen werden müssen. Die Identifizierung derartiger DNA-Sequenzen ist eine herausfordernde Forschungsaufgabe, die mit beträchtlichen Irrtümern und Ungewißheiten behaftet ist. Es besteht in immer größerem Maße ein Interesse an der Möglichkeit, Pflanzen genetisch zu modifizieren, was die damit verbundenen Aufwendungen und Bemühungen, die mit der Identifizierung transkriptionaler Sequenzen und ihrer Manipulation zu bestimmten Nutzungen verbunden sind, rechtfertigen. Es sind bereits Verfahren zur genetischen Modifikation dikotyler und monokotyler Pflanzen bekannt (EP 267 159), sowie die folgenden Publikationen von Crouch et al., In: Molecular Form and Function of the Plant Genome, eds. Van Vloten-Doting, Groot and Hall, Plenum Publishing Corp. 1985, pp 555-566; Crouch and Sussex, Planta (1981) 153:64-74; Crouch et al., J. Mol. Appl. Genet (1983) 2:273-283; und Simon et al., Plant Molecular Biology (1985) 5: 191-201, in denen verschiedene Formen von Speicherproteinen in Brassica napus beschrieben werden und von Beachy et al., EMBO J. (1985) 4:3047-3053; Sengupta-Gopalan et al., Proc. Natl. Acad. Sci. USA (1985) 82:3320-3324; Greenwood and Chrispeels, Plant Physiol. (1985) 79:65-71 and Chen et al., Proc. Natl. Acad. Sci. USA (1986) 83:8560-8564 in denen Untersuchungen, die sich mit Samenspeicherproteinen und genetischer Manipulation befassen, beschrieben werden und von Eckes et al., Mol. Gen. Genet. (1986) 205: 14-22 and Fluhr et al., Science (1986) 232:1106-1112, die die genetische Manipulation lichtinduzierter Pflanzengene beschreiben.

Es wird nun eine DNA-Sequenz einer Expressions-Kassette zur Verfügung gestellt, auf der die regulatorischen Regionen für die wundinduzierbare transkriptionale Regulation im Stengel und in Blättern sowie für die konstitutive transkriptionale Regulation in der Kartoffelknolle lokalisiert sind und die in Vektoren in das pflanzliche Genom, unter Verwendung von Agrobacterium tumefaciens als Transfer-Mikroorganismus übertragen werden kann. Die DNA-Sequenz enthält die Sequenz der regulatorischen transkriptionalen Starter-Region für die Wandinduktion und die Knollenspezifität. Dieser Sequenz der Starter-Region kann eine Sequenz nachgeschaltet werden, die die Information für die Modifikation des Phänotyps der betreffenden Zellgewebe und die Bildung sowie mengenmäßige Verteilung endogener Produkte oder die Bildung heterogener Expressionsprodukte für eine neue Funktion enthält. Zweckmäßiger Weise sollten die Transkriptions- und Terminations-Regionen in Transkriptions-Richtung durch einen Linker oder Polylinker, der eine oder mehrere Restriktionsstellen für die Insertion dieser Sequenz enthält, versehen werden.

In der Regel hat der Linker 1 bis 10, meistens 1 bis 8, vorzugsweise 2 bis 6 Restriktionsstellen. Im allgemeinen hat der Linker eine Größe von weniger als 100 bp, häufig weniger als 60 bp, mindestens jedoch 5 bp. Der transkriptionale Startbereich kann sowohl nativ bzw. homolg als auch fremdartig bzw. heterolog zur Wirtspflanze sein. Von besonderem Interesse sind die transkriptionalen Startregionen, die mit dem Kartoffel (Solanum tuberosum)-Proteinase-Inhibitor II-Gen assoziiert sind, das während der gesamten Kartoffelknollenentwicklung von der Stolonenbildung bis zur reifen Knolle exprimiert wird. Die Expression des Proteinase-Inhibitor II-Gens kann vor einer Verletzung (z.B. durch fressende Insekten), die die Expression des Proteinase-Inhibitor II-Gens in Blättern und Stengeln induziert, in anderen Pflanzenteilen nicht nachgewiesen werden. Diese wundinduzierbare Expression ist nicht nur auf verletzte Teile der Pflanze begrenzt. Eine systemische Induktion führt zu einer Akkumulation von Proteinase-Inhibitor II, sowohl in verwundeten als auch in intakten Teilen der verwundeten Pflanze. Die Transkriptions-Kassette beinhaltet in der 5'-3' Transkriptionsrichtung eine für die Pflanze repräsentative Region für die Transkription und die Translation, eine beliebige Sequenz und eine Region für die transkriptionale und translationale Termination. Der verwendete Terminationsbereich ist ein Homologes des beteiligten proteinase-Inhibitor II-Gens. Wenn ein Subfragment der proteinase-Inhibitor II Gen-Regulator-Region zu einem heterologen Promotor fusioniert ist, scheint der Terminationsbereich auztauschbar zu sein. Die DNA-Sequenz darf alle möglichen offenen Leseraster für ein beliebiges Peptid als auch ein oder mehrere Introns enthalten. Als Beispiel seien genannt: Sequenzen für Enzyme; Sequenzen, die komplementär a) zu einer Genomsequenz sind, wobei die Genomsequenz ein offener Leseraster sein darf; b) zu einem Intron; c) zu einer nicht kodierenden Leitsequenz; d) zu jeder Sequenz, die durch Komplementarität die Transkription, mRNA-Verarbeitungen, (z.B. Splicing) oder die Translation inhibiert. Die gewünschte DNA-Sequenz kann synthetisch hergestellt oder natürlich gewonnen sein oder eine Mischung aus synthetischen und natürlichen DNA-Bestandteilen enthalten. Im allgemeinen wird eine Synthetische DNA-Sequenz mit Codons erzeugt, die von Pflanzen bevorzugt werden. Diese von Pflanzen bevorzugten Codons können aus Codons mit der höchsten Proteinhäufigkeit bestimmt werden, die in den meisten interessanten Pflanzenspezies exprimiert werden. Bei der Präparation der Transkriptions-Kassette können die verschiedenen DNA-Fragmente manipuliert werden, um eine DNA-Sequenz zu erhalten, die zweckmäßiger Weise in der korrekten Richtung liest und die mit einem korrekten Leseraster ausgestattet ist. Für die Verbindungen der DNA-Fragmente untereinander können an den Fragmentenden Adapter oder Linker eingesetzt werden. Ferner können Manipulationen, die passende Restriktionsstellen bereitstellen oder die überflüssige DNA oder Restriktionsstellen entfernen, eingesetzt werden. Wo Insertionen, Deletionen oder Substitutionen, wie z.B. Transitionen und Transversionen in Frage kommen, können in vitro Mutagenese, Primerrepair, Restriktion oder Ligation verwendet werden.

Bei geeigneten Manipulationen, wie z.B. Restriktion, "chewing-back" oder Auffüllen von Überhängen für "blunt-ends" können komplementäre Enden der Fragmente für die Zusammenfügung und Ligation zur Verfügung gestellt werden. Zur Durchführung der verschiedenen Stufen ist für eine Vergrößerung der DNA-Menge und für die DNA-Analyse, die der Sicherstellung der erwarteten Erfolge der Eingriffe dient, eine Klonierung erforderlich.

Es ist eine große Anzahl Klonierungsvektoren verfügbar, die ein Replikationssystem in E.coli und einen Marker, der eine Selektion der tranformierten Zellen erlaubt, beinhalten. Die Vektoren beinhalten z.B. pBR 332, pUC-Serien, M13 mp-Serien, pACYC 184 usw. Dementsprechend kann die Sequenz an einer passenden Restriktionsstelle in den Vektor eingefügt werden. Das erhaltene Plasmid wird für die Transformation in E.coli verwendet. Die E.coli Zellen werden in einem geeigneten Nährmedium gezüchtet, anschließend geerntet und lysiert. Das Plasmid wird wiedergewonnen. Als Analysenmethoden werden im allgemeinen eine Sequenzanalyse, eine Restriktionsanalyse, Elektrophoresen und weitere biochemisch-molekularbiologische Methoden durchgeführt. Nach jeder Manipulation kann die verwendete DNA-Sequenz restriktioniert und mit der nächsten DNA-Sequenz verbunden werden. Jede Plasmid-Sequenz kann in den gleichen oder anderen Plasmiden kloniert werden. Je nach Einführungsmethode gewünschter Gene in die Pflanze können weitere DNA-Sequenzen erforderlich sein. Werden z.B. für die Transformation das Ti- oder Ri-Plasmid der Pflanzenzelle verwendet, so muß mindestens die rechte Begrenzung, häufig jedoch die rechte und die linke Begrenzung der Ti- und Ri-Plasmid T-DNA, als Flankenbereich der einzuführenden Gene, verbunden werden. Die Verwendung von T-DNA für die Transformation von Pflanzenzellen ist intensiv untersucht und ausreichend in EP 120 516; Hoekema, In: The Binary Plant Vector System Offset-drukkerij Kanters B.V., Alblasserdam, 1985, Chapter V; Fraley, et al., Crit. Rev. Plant Sci., 4:1-46 und An et al., EMBO J. (1985) 4:277-284 beschrieben worden.

Ist die eingeführte DNA erst einmal im Genom integriert, so ist sie dort auch relativ stabil und springt in der Regel nicht mehr heraus. Sie enthält normalerweise einen Selektionsmarker, der den transformierten Pflanzenzellen Resistenz gegenüber einem Biozid oder einem Antibiotikum wie Kanamycin, G 418, Bleomycin, Hygromycin oder Chloramphenicol u.a. vermittelt. Der individuell verwendete Marker sollte daher die Selektion transformierter Zellen gegenüber Zellen, denen die eingefügte DNA fehlt, gestatten.

Für die Einführung von DNA in eine pflanzliche Wirtszellen stehen eine Vielzahl von Techniken zur Verfügung. Diese Techniken umfassen die Transformation mit T-DNA unter Verwendung von Agrobacterium tumefaciens oder Agrobacterium rhizogenes als Transformationsmittel, die Fusion, die Injektion oder die Elektroporation sowie weitere Möglichkeiten. Werden für die Transformation Agrobakterien verwendet, muß die einzuführende DNA in spezielle Plasmide kloniert werden und zwar entweder in einen intermediären Vektor oder einen binären Vektor. Die intermediären Vektoren können aufgrund von Sequenzen, die homolog zu Sequenzen in der T-DNA sind, durch homologe Rekombination in das Ti-oder Ri-Plasmid integriert werden. Dieses enthält außerdem die für den Transfer der T-DNA notwendige Vir-Region. Intermediäre Vektoren können sich nicht in Agrobakterien replizieren. Mittels eines Helferplasmids kann der intermediäre Vektor auf Agrobacterium tumefaciens übertragen werden (Konjugation). Binäre Vektoren können sich in E.coli als auch in Agrobakterien replizieren. Sie enthalten ein Selektionsmarker-Gen und einen Linker oder Polylinker, welche von der rechten und linken T-DNA Grenzregion eingerahmt werden. Sie können direkt in die Agrobakterien transformiert werden (Holsters et al., Mol. Gen. Genet. (1978), 163:181-187). Das als Wirtszelle dienende Agrobakterium soll ein Plasmid, das die Vir-Region trägt, welche für den Transfer der T-DNA in die Pflanzenzelle notwendig ist, enthalten, wobei zusätzliche T-DNA enthalten sein kann. Das so transformierte Bakterium wird zur Transformation von Pflanzenzellen benutzt. Für den Transfer der DNA in die Pflanzenzelle können Pflanzen-Explanate zweckmäßiger Weise mit Agrobacterium tumefaciens oder Agrobacterium rhizogenes kultiviert werden. Aus dem infizierten Pflanzenmaterial (z.B. Blattstücke, Stengelsegmente, Wurzeln, aber auch Protoplasten oder Suspensions-kultivierte Zellen) können dann in einem geeigneten medium, welches Antibiotika oder Biozide zur Selektion enthalten kann, wieder ganze Pflanzen regeneriert werden, die dann auf die Anwesenheit der eingeführten DNA getestet werden können. Bei der Injektion und Elektroporation sind keine speziellen Anforderungen an die Plasmide gestellt, es können einfache Plasmide, wie z.B. pUC-Derivate, benutzt werden. Für die Einführung fremder Gene in die Pflanzen steht eine Vielzahl von Möglichkeiten offen, besonders interessant ist jedoch die Expression Schädlingsresistenz-vermittelnder Gene, die nach einer Verwundung der Pflanze zu einer reduzierten Nährstoffqualität der Pflanze für Schädlinge oder für eine Schädlingstoxizität sorgen. Dadurch wird ein übermäßiger Insektenfraß vermieden, was zu einem höheren Ernteertrag führt. Die entsprechenden Gene können Toxin-Gene sein, die z.B. für B. thuringiensis δ-Endotoxin kodieren oder Gene sein, die für Insektenhormone, die zu einem Wachstumswechsel von Insektenlarven führen (z.B. Ekdyson), kodieren.

Alternativ können Gene für verschiedensten Ausgangsprodukte, einschließlich Säugerprodukte, wie z.B. Blutfaktoren; Lymphokine; Koloniestimulationsfaktoren; Interferone; Plasminogen-Aktivatoren; Enzyme, wie z.B. Superoxid-Dismutase oder Chymosin; Hormone; Thioesterase-2 aus Rattenmilch; Phospholipid-Acyl-Desaturase, die an der Synthese von Cicosapentaenoia-Säure beteiligt ist oder Human-Serum-Albumin, verwendet werden. Eine weitere Möglichkeit ist die Erhöhung der Menge an Knollenproteinen, insbesondere mutierte Knollenproteine, die eine optimierte Aminosäurezusammensetzung (essentielle Aminosäuren) aufweisen, und auf diese Weise den Nährwert der Knollen steigern können. Soll die Menge bestimmter endogener Produkte verringert werden, ist außerdem die Expression der Gene oder von Teilen dieser Gene in falscher Orientierung zum Promotor denkbar, was zur Synthese einer RNA führt, die komplementär zu einem gesamten oder zu Teilen eines endogenen Gens ist und dadurch die Transkription dieses Gens oder die Prozessierung und/oder Translation der endogenen mRNA hemmen kann.

Die transformierten Zellen wachsen innerhalb der Pflanzen in der üblichen Weise (siehe auch McCormick et al., Plant Cell Reports (1986) 5, 81-84). Diese Pflanzen können normal angezogen werden und mit Pflanzen, die die gleiche transformierte Erbanlage oder andere Erbanlagen besitzen, gekreuzt werden. Die daraus entstehenden hybriden Individuen haben die entsprechenden phänotypischen Eigenschaften. Es sollten zwei oder mehrere Generationen angezogen werden, um sicherzustellen, daß das phänotypische Merkmal stabil beibehalten und vererbt wird. Auch sollten Samen geerntet werden, um sicherzustellen, daß der entsprechende Phänotyp oder andere Eigenerten enthalten sind. Als Wirtspflanze für die wundinduzierbare Expression kann jede Pflanzenart, die von wirtschaftlichem Interesse ist, verwendet werden. Für die knollenspezifische Expression ist Solanum tuberosum geeignet.

Die Identifizierung brauchbarer transkriptionaler Startbereiche kann auf einer Vielzahl von Wegen erreicht werden. Man bedient sich hier in der Regel der mRNAs, die aus bestimmten Teilen der Pflanze (Knolle) oder während bestimmter Zustände der Pflanze (nicht-verwundet / verwundet) isoliert worden sind. Für die zusätzliche Konzentrationssteigerung der spezifisch an das Gewebe oder den pflanzlichen Zustand assoziierten mRNA kann cDNA präpariert werden, wobei unspezifische cDNA an der mRNA oder der cDNA aus anderen Geweben oder Pflanzenzuständen (z.B. verwundet / nicht verwundet) abgezogen werden kann. Die restliche cDNA kann dann für die Sondierung des Genoms der Komplementärsequenzen, unter Benutzung einer geeigneten Pflanzen-DNA-Bibliothek verwendet werden. Wo das Protein isoliert ist oder isoliert wird, kann es partiell sequenziert werden, so daß eine Sonde zur direkten Identifizierung der entsprechenden Sequenzen in einer Pflanzen-DNA-Bibliothek hergestellt werden kann. Anschließend können die Sequenzen, die mit der Sonde hybridisieren, isoliert und manipuliert werden. Ferner kann dr nicht translatierte 5´-Bereich, der mit dem kodierenden Bereich assoziiert ist, isoliert und in Expressions-Kassetten für die Identifizierung der transkriptionalen Aktivität des nicht translatierten 5´-Bereichs verwendet werden. Die gewonnenen Expressions-Kassetten, die die nicht übersetzte 5´-Region verwenden, können in Pflanzen transformiert werden (s.o.), um ihre Funktionsfähigkeit mit einem heterologen Strukturgen (anders als der offene Leseraster des Wildtyps, der mit der nicht übersetzten 5´-Region assoziiert ist) sowie die Knollen- und Wundspezifität zu prüfen. Auf diese Weise können spezifische Sequenzen, die für die knollen- und wundspezifische Transkription notwendig sind, identifiziert werden. Expressions-Kassetten, die von besonderem Interesse sind, enthalten transkriptionale Initionsstellen der Protein-Inhibitor II-Gene.

Begriffe und Abkürzungen

Abkürzungen

bp = Basenpaare
DNA = Desoxyribonukleinsäure, Träger der genetischen Information
cDNA = Durch Reverse - Transkriptase hergestellte Kopie einer mRNA mRNA = Messenger (Boten) - Ribonukleinsäure T-DNA = Transfer-DNA (auf dem Ti-Plasmid von Agrobacterium tumefaciens lokalisiert)

Begriffe

blunt-ends = DNA-Enden, in denen beide DNA-Stränge exakt gleich lang sind.

chewing-back = enzymatische Entfernung von Nukleotiden eines DNA-Stranges, der länger als der komplementäre Strang eines DNA-Moleküls ist.
Elektrophorese = biochemisches Trennverfahren zur Trennung von Proteinen und Nukleinsäuren nach Größe und Ladung.
Endonuklease = Enzym, das unabhängig vom Kettenende spaltet.
Exonuklease = Enzym, das nur am Ende einer Polynukleotidkette angreifen kann.
Expression = Aktivität eines Gens.
Gen = Erbfaktor; eine Einheit des Erbguts, Träger der Teilinformation für ein bestimmtes Merkmal. Gene bestehen aus Nukleinsäuren (z.B. DNA, RNA).
Genom = Gesamtheit der auf den Chromosomen der Zelle lokalisierten Gene.
Genom-Sequenz = DNA-Sequenz des Genoms, wobei drei hintereinanderliegende Nukleotidbasen (Triplett) ein Codon bilden, welches wiederum für eine bestimmte Aminosäure kodiert.
RNA-Splicing = ein Gen liegt nicht immer als kolineare Einheit vor, sondern kann nichtkodierende Sequenzen (Introns) enthalten, die aus der mRNA herausgespalten (Splicing) werden müssen.
heterologe(s) Gen(e) oder DNA = Fremd-Gen oder Fremd-DNA
homologe(s) Gen(e) oder DNA = Gen oder DNA stammt aus der gleichen Spezies.
Klenow-Enzym = durch Spaltung mit Subtilisin erhaltenes 76.000 D großes Fragment der DNA-Polymerase I. Besitzt 5´ - 3´ Polymerase -und 3´ - 5´ Exonuclease-Aktivität, aber nicht die 5´ - 3´ - Exonuklease-Aktivität des Holoenzyms.
Klon = Zellpopulation, die von einer einzigen Mutterzelle stammt. Die Nachkommen sind genotypisch gleich. Durch Klonierung kann die Einheitlichkeit von Zellinien noch gesteigert werden.
Ligation = enzymatische Bildung einer Phosphodiesterbindung zwischen 5´-Phosphatgruppen und 3´-Hydroxylgruppen der DNA.
Linker, Polylinker = synthetische DNA-Sequenz, die eine oder mehrere (Polylinker) Restriktionsschnittstellen in unmittelbarer Reihenfolge enthält.
Northern Blots, Southern Blots = Transfer und Fixierung elektrophoretisch aufgetrennter RNA Southern Blots oder DNA auf eine Nitrozellulose- oder Nylonmembran.
Phänotyp = Summe der Merkmale, die in einem Organismus im Gegensatz zu seiner Genausstattung (Genotyp) ausgeprägt ist.
Plasmid = zusätzlicher, extrachromosomaler DNA-Erbträger in Bakterienzellen (evtl. auch in Eukaryonten), der sich unabhängig vom Bakterienchromosom redupliziert. Das Plasmid kann in andere Wirts-DNA integriert werden.
Primer = Starstück; Polynukleotidstrang, an dem weitere Nukleotide angehängt werden können.

Promotor = Kontrollsequenz der DNAExpression, die die Transkription homologer oder heterologer DNA-Gen-Sequenzen gewährleistet.

Regulator-Proteine = Proteine, die mit DNA in Wechselwirkung stehen und die Genexpression steuern.

Replikation = Verdopplung der DNA-Sequenz

Restriktionsenzyme = Restriktionsendonukleasen, die eine Untergruppe der Endodesoxyribonukleasen sind (z.B. EcoRI (Spezifität G↓AATTC und Eco-RII↓CC($^A_T$) GG, aus E.coli), zeichnen sich durch eine hohe Spezifität der Substraterkennung aus (↓= Spaltstelle).

Restriktionsstellen = Spaltstellen, die spezifisch durch Restriktionsenzyme erzeugt werden.

Termination = letzte Stufe der Protein- bzw. der RNA-Synthese.

Transformation = Einfügung exogener DNA eines Bakterienstammes in eine Empfängerzelle.

Transkription = Überschreibung der auf der DNA enthaltenen genetischen Information auf eine RNA.

Translation = Übersetzung der genetischen Information, die in Form einer linearen Abfolge von Basen in Nukleinsäuren gespeichert ist. Das Produkt der Übersetzung ist ein Polyeptid, das aus einer Abfolge von Aminosäuren besteht.

Transition = Basenpaaraustausch: Purin-Pyrimidin zu Purin-Pyrimidin, z.B. A-T durch G-C

Transversion = Basenpaaraustausch: Purin-Pyrimidin zu Pyrimidin-Purin, z.B. A-T durch T-A

Deletion = Entfernung eins oder mehreren Basenpaare

Insertion = Einbau eins oder mehrerer Basenpaare;

Transition, Transversion, Deletion und Insertion sind Punktmutationen.

Transposom = eine Einheit, bestehend aus Resistenzgen(en) und zwei IS-Elementen (IS = integrated segments)

IS-Elemente oder IS-Sequenzen sind DNA-sequenzen, die die Expression benachbarter Gene beinflussen können, Deletionen induzieren können und die Eigenart besitzen, sich in das Bakteriengenom an verschiedenen Stellen ein- und auch wieder auszubauen.

Vektoren = wirtsspezifische, replizierfähige Strukturen, die Gene aufnehmen und diese auf andere Zellen übertragen. Plasmide können als Vektoren benutzt werden.

Am 16.12.1988 wurde bei der Deutschen Sammlung von Mikroorganismen (DSM) in Braunschweig, Bundesrepublik Deutschland, folgender Mikroorganismus hinterlegt (Hinterlegungsnummer):

Agrobacterium tumefaciens A.tum. M 14, enthaltend den Vektor pM 14 (DSM 5088)

Beschreibung der Abbildung

Abb. 1

Zeigt den Vektor pM 14, auf dem die 3,4 kb lange EcoRI/HindIII-DNA-Sequenz lokalisiert ist. Diese DNA-Sequenz enthält das 1,3 kb lange ScaI/HindIII-Fragment des Proteinase-Inhibitor II-Promotors, das 1,8 kb lange BamHI/SstI-Fragment der β-Glucuronidase und das 0,26 kb lange SphI/SphI-Fragment des Proteinase-Inhibitor II. Es sind ferner die Schnittstellen sowie die Stelle des Transkriptionsstarts eingetragen.

Zum besseren Verständnis der dieser Erfindung zugrundeliegenden Ausführungsbeispiele wird vorab eine Aufstellung aller für diese Versuche notwendigen und an sich bekannten Verfahren gegeben:

1. Klonierungsvektoren

Zur Klonierung wurden die Vektoren pUC18/19 und M13mp18/19 (Yanisch-Perron et al., Gene (1985), 33, 103-119) benutzt.

Für die Pflanzentransformation wurden die Genkonstruktionen entweder in den intermediären Vektor pMPK110 (Eckes, Doktorarbeit (1985) - Standort der Arbeit - Universitätsbibliothek Köln) oder den binären Vektor BIN19 (Bevan, Nucl. Acids. Res. (1984), 12, 8711-8720) kloniert.

2. Bakterienstämme

Für die pUC- und M13-Vektoren wurden die E.coli-Stämme BMH71-18 (Messing et al., Proc. Nat. Acad. Sci. USA (1977), 24, 6342-6346) oder TB1 benutzt. Für die Vektoren pMPK110 und BIN19 wurde ausschließlich der Stamm TB1 benutzt. TB1 ist ein Rekombinations-negatives, Tetracyclin-resistentes Derivat des Stammes JM101 (Yanisch-Perron et al., Gene (1985), 33, 103-119). Der Genotyp des TB1-Stammes ist (Bart Barrel, pers. Mitteilung): F' (traD36, proAB, lacI, lacZΔM15), Δ(lac, pro), SupE, thiS, recA, Sr1::Tn10(Tc$^R$).

Als Helferstamm für den konjugativen Transfer der pMPK-Plasmide aus den TB1-Zellen in Agrobacterium tumefaciens diente der E.coli-Stamm GJ23 (Van Haute et al., EMBO J. (1983), 2, 411-417).

Die Pflanzentransformation wurde mit Hilfe der Agrobacterium tumefaciens-Stämme GV3850Kan (Jones et al., EMBO J. (1985), 4, 2411-2418, pMPK-Plasmid) bzw. GV2260 (Deblaere et al., Nucl, Acids Res. (1985), 13, 4777-4788; Bin19-Derivate) durchgeführt.

Medien:

YT-Medium: 0,5 % Hefe-Extrakt, 0.5 % NaCl, 0.8 % Bacto-Trypton, evtl. 1,5 % Agar
YEB-Medium: 0,5 % Rindfleisch-Extrakt, 0,1 % Hefe-Extrakt, 0,5 % Pepton, 0,5 % Saccharose, 2 mM MgSO4, evtl. 1,5 % Agar
MS-Medium: nach Murashige und Skoog (Physiologia Plantarum (1962), 15, 473-497)

3. Konjugation in bzw. Transformation von Agrobacterium tumefaciens

Für den Transfer der rekombinanten pMPK110-Plasmide wurden jeweils 20 μl einer unter Selektion gewachsenen Übernachtkultur der pMPK110-enthaltenden TB1-Zellen (50 μg/ml Spectinomycin und 25 μg/ml Streptomycin in YT-Medium), des Helferstammes GJ23 (10 μg/ml Tetracyclin und 25 μg/ml Kanamycin in YT-Medien) und des Agrobacterium tumefaciens-Stammes GV3850Kan (50 μg/ml Erythromycin und 50 μg/ml Chloramphenicol in YEB-Medium) miteinander gemischt und auf einer YEB-Agarplatte ohne Selektion über Nacht bei 28 °C inkubiert. Der hierbei entstandene Bakterienrasen wurde dann auf YEB-Platten, die 50 μg/ml Chloramphenicol, 50 μg/ml Erythromycin, 100μg/ml Spectinomycin und 300 μg/ml Streptomycin enthielten, ausplattiert. Die nach 3tägiger Inkubation bei 28 °C gewachsenen Kolonien wurden nun bei gleicher Selektion auf YEB-Platten vereinzelt. Aus hierbei entstandenen Einzelkolonien wurde die Gesamt-DNA isoliert, die nach geeigneter Restriktionsspaltung mit Hilfe von "Southern Blots" auf die Integration der rekombinaten DNA untersucht wurde.

Bei Bin19-Derivaten erfolgte die Einführung der DNA in die Agrobakterien durch direkte Transformation nach der Methode von Holsters et al. (Mol. Gen. Genet. (1978), 163, 181-187). Die Plasmid-DNA transformierter Agrobakterien wurde nach der Methode von Birnboim und Doly (Nucl. Acids Res. (1979), 7, 1513-1523) isoliert und nach geeigneter Restriktionsspaltung gelelektrophoretisch aufgetrennt.

4. Pflanzentransformation

A) Tabak: 10 ml einer unter Selektion gewachsen Übernachtkultur von Agrobacterium tumefaciens wurden abzentrifugiert, der Überstand verworfen, und die Bakterien im gleichen Volumen Antibiotika-freien Mediums resuspendiert. In einer sterilen Petrischale wurden Blattscheiben steriler Pflanzen (ca. 1 cm²), denen die Mittelrippe entfernt wurde, in dieser Bakteriensuspension gebadet. Anschließend wurden die Blattscheiben in Petrischalen, die MS-Medium mit 2 % Saccharose und 0,8 % Bacto-Agar enthalten, dicht ausgelegt. Nach 2tägiger Inkubation bei 25 °C im Dunkeln wurden sie auf MS-Medium übertragen, welches 100 mg/l Kanamycin, 500 mg/l Claforan, 1 mg/l Benzylaminopurin (BAP), 0,2 mg/l Naphthylessigsäure (NAA) und 0,8 % Bacto-Agar enthielt. Wachsende Sprosse wurden auf hormonfreies MS-Medium mit 250 mg/l Claforan überführt und auf Nopalingehalt geprüft (Otten et al., Biochimica et Biophysica Acta (1978), 527, 497-500). Positive Sprosse wurden nach Wurzelbildung in Erde überführt.

B) Kartoffel: 10 kleine, mit einem Skalpell verwundete Blätter einer Kartoffel-Sterilkultur wurden in 10 ml MS-Medium mit 2 % Saccharose gelegt, welches 30-50 μl einer unter Selektion gewachsenen Agrobacterium tumefaciens-Übernachtkultur enthielt. Nach 3-5 minütigem, leichtem Schütteln wurden die Petrischalen bei 25 °C im Dunkeln inkubiert. Nach 2 Tagen wurden die Blätter auf MS-Medium mit 1,6 % Glukose, 2 mg/l Zeatinribose, 0,02 mg/l Naphthylessigsäure, 0,02 mg/l Giberellinsäure, 500 mg/l Claforan, 50 mg/l Kanamycin und 0,8 % Bacto-Agar ausgelegt. Nach einwöchiger Inkubation bei 25 °C und 3000 Lux wurde die Claforankonzentration im Medium um die Hälfte reduziert.

5. Analyse genomischer DNA aus transgenen Pflanzen

Die Isolierung genomischer Pflanzen-DNA erfolgte nach Rogers und Bendich (Plant Mol. Biol. (1985), 5, 69-76).
Für die DNA-Analyse wurden 10-20 μg DNA nach geeigneter Restriktionsspaltung mit Hilfe von "Southern Blots" auf Integration der zu untersuchenden DNA-Sequenzen untersucht.

6. Analyse der Gesamt-RNA aus transgenen Pflanzen

Die Isolierung pflanzlicher Gesamt-RNA erfolgte nach Logemann et al. (Analytical Biochem. (1987), 163, 16-20).
Für die Analyse wurden je 50 μg Gesamt-RNA mit Hilfe von Northern Blots auf die Anwesenheit der gesuchten Transkripte untersucht.

7. CAT-Test

Die Aktivität der Chloramphenicol-Acetyltransferase (CAT) in transgegen Pflanzen wurde nach Colot et al. (EMBO J. (1987), 6, 3559-3564) durchgeführt. Die Bradford-Proteinbestimmung erfolgte jedoch vor der Hitzebehandlung (75 °C, 10 min.) des Extraktes. Für die Bestimmung der CAT-Aktivität wurde eine Menge des hitzebehandelten Extraktes eingesetzt, die einem Proteingehalt von 500 µg des gemessenen Extraktes entsprach.

## 8. GUS-Test

Die Aktivität der ß-Glucuronidase (GUS) in transgenen Pflanzen wurden nach der Methode von Jefferson (Plant Mol. Biol. Rep. (1987), 5, 387-405) bestimmt. Die Proteinbestimmung erfolgte wie beim CAT-Test nach Bradford (Anal. Biochem. (1976), 72, 248-254). Für die Bestimmung der GUS-Aktivität wurden 50 µg Protein eingesetzt, die Inkubation erfolgte bei 37 °C für 30 Minuten.

Die nachfolgenden Ausführungsbeispiele erläutern die Isolierung und Identifizierung sowie die Funktion und Verwendung des wundinduzierbaren und knollenspezifischen proteinase-Inhibitor II-Promotors aus Kartoffelknollen.

## Ausführungsbeispiel 1

Klonierung und Strukturanalyse eines Proteinase-Inhibitor II-Gens aus Solanum tuberosum

cDNA-Klone, die für den Proteinase-Inhibitor II der Kartoffel kodieren, wurden aus der Kartoffel-Varietät "Berolina" isoliert und sequenziert (Sanchez-Serrano et al., Mol. Gen, Genet (1986), 203, 15-20). Diese cDNA-Klone dienten dazu, einen homologen, genomischen Proteinase-Inhibitor II-Klon aus der monohaploiden Kartoffelline AM 80/5793 (Max-Planck-Institut für Züchtungsforschung, Köln) zu isolieren; durch Restriktions- und Sequenzanalyse wurde die genaue Struktur des Gens bestimmt, ferner konnte der Transkriptionssart durch RNase-Verdauung eines SP6-Antisense-RNA/mRNA-Hybrids ermittelt werden (Keil et al., Nucl. Acids Res (1986), 14, 5641-5650).

## Ausführungsbeispiel 2

Identifizierung der für die Wundinduzierbarkeit des Proteinase-Inhibitor II-Gens verwortlichen regulatorischen Bereiche

Es konnte gezeigt werden, daß das isolierte Proteinase-Inhibitor II-Gen (s. Ausführungsbeispiel 1) in transgenen Wisconsin 38-Tabakpflanzen, die selber keine zum Proteinase-Inhibitor II-Gen homologen Sequenzen enthalten, durch Verwundung in Blättern und im Stengel induzierbar ist (Sanchez-Serrano et al., EMBO J. (1987), 6, 303-306). Das DNA-Fragment, welches dabei in die Pflanzen eingeführt worden ist, reicht von einer EcoRI-Restriktionsschnittstelle, die sich ca. 3 kb 5' vor dem Transkriptionsstart des Proteinase-Inhibitor II-Gens befindet, bis zu einer EcoRI-Schnittstelle, die ca. 1,5 kb 3' hinter der vermutlichen Polyadenylierungsstelle lokalisiert ist.

In einem anderen Experiment wurde das Proteinase-Inhibitor II-Gen nach Deletion des einzigen Introns durch Austausch der Intron-enthaltenden genomischen Sequenzen durch die entsprechenden cDNA-Sequenzen in Tabak (Wisconsin 28) eingeführt. In diesem Fall wurde eine HindII-Schnittstelle 1,5 Kb 5' vor dem Transkriptionsstart benutzt, die EcoRI-Schnittstelle 1,5 Kb 3' hinter dem Gen wurde beibehalten. Die Analyse der mRNA der mit dieser Konstruktion erhaltenen transgenen Tabakpflanzen ergab eine Wundinduzierbarkeit des intronlosen Proteinase-Inhibitor II-Gens, die mit der des entsprechenden intronhaltigen Gens vergleichbar ist. So scheint das Intron des Proteinase-Inhibitor II-Gens keine für die Wundinduzierbarkeit notwendigen, regulatorischen Elemente zu enthalten.

## Ausführungsbeispiel 3

Promotor-Deletionskonstruktionen

Von den Ergebnissen aus Ausführungsbeispiel 2 ausgehend wurden chimäre Konstruktionen von durch Exonuklease III-Verdauung erhaltenen Deletions- als auch Restriktionsfragmenten des Proteinase-Inhibitor II-Gens mit dem bakteriellen Chloramphenicol-Acetyltransferase-Gen (CAT) und der 3'-Region des Proteinase-Inhibitor II-Gens hergestellt.

Die Deletionsfragmente stammen aus der Sequenzanalyse des Proteinase-Inhibitor II-Gens. Sie wurden mit Hilfe einer EcoRI-Schnittstelle, die sich 5' vor den entsprechenden Promotordeletionen im Polylinker des Phagenvektors M13mp19 befindet, und einer Scal-Schnittstelle 32 bp 3' vom Transkriptionsstart und 18 bp 5' vom ATG-Startkodon des Proteinase-Inhibitor II-Gens in den EcoRI/Smalgespaltenen intermediären Vektor pMPK110 kloniert. Die auf diese Weise erhaltenen Promotorfragmente hatten eine Länge von 700, 514, 210 und 150 bp 5' vom Transkriptionsstart des Proteinase-Inhibitor II-Gens.

Als Restriktionsfragment diente einerseits ein Fragment, das von einer HindIII-Schnittstelle 1,3 kb 5' vor dem Transkriptionsstart bis zu der oben erwähnten Scal-Schnittstelle reicht (pM 11), als auch ein 441 bp langes SspI/ScaI-Fragment der Promotorregion, die jeweils in die SmaI-Schnittstelle von pMPK110 kloniert wurden. Die HindIII-Schnittstelle des ersten Fragmentes mußte dazu zuerst mit T4-DNA-Polymerase aufgefüllt werden.

Das CAT-Gen wurde als 800 bp langes BamHI-Fragment (Velten et al., Nucl. Acids Res. (1985), 13, 6981-6998) jeweils 3' hinter die Promotorfragmente des Proteinase-Inhibitor II-Gens in die BamHI-Schnittstelle des Polylinkers von pMPK110 kloniert.

Als Polyadenylierungssignal wurde das mit SphI-Linkern versehene 260 bp lange RsaI/SspI-Fragment des Proteinase-Inhibitor II-Gens in die entsprechende Restriktionsschnittstelle des Polylinkers von pMPK110 kloniert. Die RsaI-Schnittstelle befindet sich 11 bp vor dem TGA-Stopkodon, die SspI-Schnittstelle befindet sich 74 bp 3' hinter der Polyadenylierungsstelle des Proteinase-Inhibitor II-Gens.

Diese Konstruktion wurde mit Hilfe des Agrobacterium tumefaciens-Tranformationssystems in die Tabak-Varietät Wisconsin 38 eingeführt. Von den erhaltenen transgenen Tabakpflanzen wurden jeweils nicht-verwundete und verwundete Blätter auf die Anwesenheit von CAT-mRNA bzw. auf die Aktivität des CAT-Enzyms getestet. Die Ergebnisse zeigen, daß für die maximale Wundinduzierbarkeit der Bereich von 700 - 1300 bp 5' vor dem Transkriptionsstart notwendig ist (pM 11). Nach Deletion dieses Bereiches ist, wenn überhaupt, nur eine sehr schwache Wundinduzierbarkeit zu beobachten. Intersssanterweise konnte keine CAT-Aktivität mit einer Konstruktion beobachtet werden, die zwar den gesamten Promotor des Proteinase-Inhibitor II-Gens bis 1500 bp 5' vor dem Transkriptionsstart besaß, aber nicht das 3'-Ende des Proteinase-Inhibitor II-Gens. Anstelle dessen wurde in dieser Konstruction 3' hinter dem Promotor ein 1000 bp langes SalI-Fragment in die entsprechende Schnittstelle des pMPK110-Polylinkers kloniert, welches 3' hinter dem CAT-Gen das Polyadenylierungssignal des Gens 7 der T-DNA von Agrobacterium tumefaciens enthält (Velten et al., Nucl. Acids Res. (1985), 13, 6981-6998). Dieses Polyadenylierungssignal ist in Pflanzenzellen funktional.

Somit läßt sich zusammenfassen, daß die regulatorischen Bereiche des Proteinase-Inhibitor II-Gens auch in Kombination mit einem heterologen, bakteriellen Gen, in diesem Fall der Chloroamphenicol-Acetyltransferase, in spezifischer Weise funktionieren. Für maximale Wundinduzierbarkeit des chimären Gens ist ein Enhancer notwendig, der sich im Bereich von 700 - 1300 bp 5' vor dem Transkripsionsstart befindet, als auch ein 260 bp langes Fragment der 3'-Region des Proteinase-Inhibitor II-Gens, welches sich bis 74 bp 3' hinter die Polyadenylierungsstelle erstreckt.

Ausführungsbeispiel 4

Fusionen eines Proteinase-Inhibitor II-Promotorfragments mit einem heterologen Promotor

Sollte der Proteinase-Inhibitor II-Promotor tatsächlich einen Enhancer enthalten, müßte dieser in der Lage sein, einen inaktiven Promotor, der lediglich die TATA- und CAAT-Steuerelemente enthält, zu aktivieren.

Dazu wurde zunächst eine CAT-Konstruktion mit einem inaktiven Promotor hergestellt. Der in Pflanzenzellen konstitutiv und sehr stark exprimierte 35 S-Promotor des Cauliflower Mosaikvirus wurde als 550 bp langes EcoRI/KpnI-Fragment in die entsprechenden Restriktionsschnittstellen vor das 1000 bp lange SalI-Fragment des CAT-Gens mit dem Gen 7-Polyadenylierungssignal in pUC18 kloniert (pDHCAT1). Die KpnI-Schnittstelle befindet sich an Position + 10 bezüglich des Transkriptionsstarts des 35 S-Promotors. Die EcoRV-Schnittstelle an Position -90 bezüglich des Transkriptionsstarts des 35 S-Promotors und die HincII-Schnittstelle 3' hinter dem Gen 7-Polyadenylierungssignal wurde ausgenutzt, um das resultierende (-90)-35S/CAT/g7pA-Fragment in die HincII-Schnittstelle von pMPK110 zu klonieren (pMP35SCAT1). Dazu war eine Partialverdauung von pDHCAT1 mit HincII notwendig, da 5' vor dem CAT-Gen eine zweite HincII-Schnittstelle vorhanden ist, die ungespalten bleiben mußte. Um die 5' vor dem (-90)35 S-Promotor localisierte SmaI-Schnittstelle für die Insertion von DNA-Fragmenten benutzen zu können, wurde die SmaI-Schnittstelle unmittelbar 5' vor dem CAT-Gen eliminiert. Dazu wurde pMP(-90)-35SCAT1 mit SalI gespalten, die Schnittstelle mit Klenow-Enzym aufgefüllt, und anschließend mit PstI gespalten. Die Ligation mit SmaI/PstI-gespaltenem pDHCAT1 ergab pMP(-90)35SCAT10, in dem die SalI- und die SmaI-Schnittstellen, die sich zwischen dem (-90)35 S-promotor und dem CAT-Gen befinden, fusioniert und somit nicht mehr benutzbar sind. Diese Konstruktion wurde in Wisconsin 38-Tabakpflanzen eingeführt; in den dadurch erhaltenen transgegen Pflanzen konnte weder in nicht-verwundeten noch in verwundeten Blättern eine Aktivität des (-90)35 S-Promotors nachgewiesen werden.

Um die Frage beantworten zu können, ob der Proteinase-Inhibitor II-Promotor diesen inaktiven (-90)35 S-Promotor aktivieren kann, wurde ein bei der Sequenzanalyse des Proteinase-Inhibitor II-Gens resultierendes, in M13mp19 kloniertes Deletionsfragment 5' vor diesen Promotor kloniert. Das Deletionsfragment, welches von Position -195 bis -1300 bezüglich des Transkriptionsstarts des Proteinase-Inhibitor II-Gens reicht, wurde durch eine EcoRI/HindIII-Spaltung aus dem M13mp19-Vektor herausgespalten. Nach Auffüllen der Schnitttellen mit T4-DNA-Polymerase wurde dieses Fragment in die SmaI-Schnittstelle von pMP(-90)35 SCAT10 insertiert. Die resultierenden Vektoren pM21 und pM22 enthalten des Promotorfragment des Proteinase-Inhibitor II-Gens in beiden Orientierungen.

Die Analyse der mit diesen Konstruktionen erhaltenen transgenen Tabakpflanzen ergab sowohl im Northern Blot als auch im CAT-Test eine deutliche Wundinduzierbarkeit des CAT-Gens in Blättern.

Dieses Resultat zeigt, daß der Proteinase-Inhibitor II-Promotor tatsächlich einen Enhancer besitzt, der in beiden Orientierungen einen inaktiven Promotor spezifisch aktivieren kann. Ferner wird deutlich, daß der der Bereich von +32 bis -195 des Proteinase-Inhibitor II-Gens nicht für die Wundinduzierbarkeit notwendig ist. Interessanterweise ist in der Kombination des (-1300/-195)-Promotorfragments des Proteinase-Inhibitor II-Gens mit dem (-90)35 S-Promotor die 3'-Region des Proteinase-Inhibitor II-Gens nicht notwendig, um Wundinduzirbarkeit des CAT-Gens zu erhalten, wie es für den gesamten Proteinase-Inhibitor II-promotor von Position -1300 bis +32 bezüglich des Transkriptionsstarts zutrifft.


Ausführungsbeispiel 5

Fusionen der regulatorischen Bereiche des Proteinase-Inhibitor II-Gens mit einem anderen bakteriellen Gen

Um zu zeigen, daß die regulatorischen bereiche des Proteinase-Inhibitor II-Gens auch in Kombination mit anderen Genen funktionieren, wurde neben der Fusion mit dem Gen für die bakterielle Chloramphenicol-Acetyltransferase eine Fusion mit dem Gen, welches für die bakterielle ß-Glucuronidase (GUS) kodiert, konstruiert.

Das GUS-Gen wurde zunächst auch dem Vektor pBI101 (Jefferson et al., EMBO J. (1987), 6, 3901-3907), zusammen mit dem Polyadenylierungssignal des Nopalinsynthase-Gens (Nos) von Agrobacterium tumefaciens, durch eine EcoRI/SmaI-Spaltung ausgeschnitten und nach Klenow-Auffüllung der Restriktionsschnittstellen in die HincII-Schnittstelle von pUC18 kloniert (pGUS; Meike Köster, pers. Mitteilung).

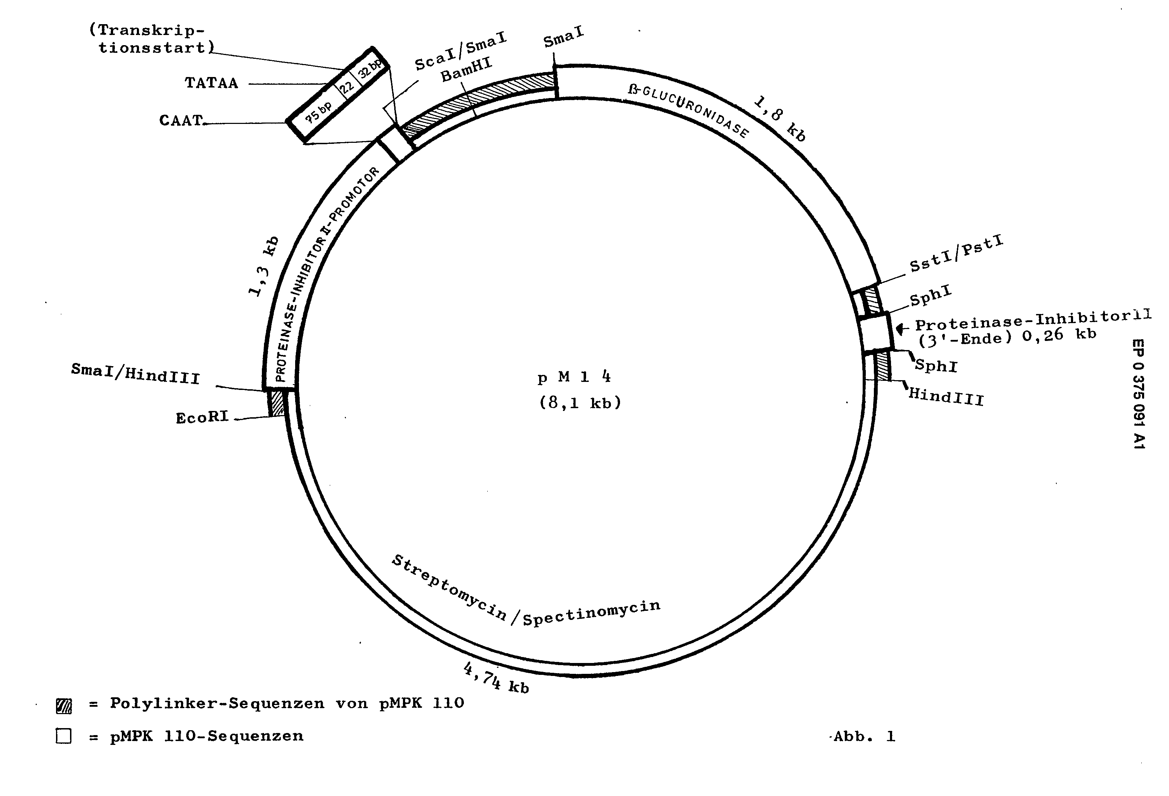

Durch eine BamHI/PstI-Spaltung wurde das CAT-Gen aus pM11 herausgespalten. Anstelle dessen wurde in den Vektor ein 1800 bp langes BamHI/SstI-Fragment aus dem Plasmid pGUS, welches das Gen für die ß-Glucuronidase ohne das Nos-3'-Ende enthält, ligiert. Dazu mußten die PstI- und die SstI-Schnittstellen vorher mit T4-DNA-Polymerase aufgefüllt werden. Der resultierende Vektor pM14 (s. Abb. 1) enthält nun das GUS-Gen 3'

hinter dem Proteinase-Inhibitor II-Promotor und 5' vor dem 3'-Ende des Proteinase-Inhibitor II-Gens (s. Abb. 1). Da die Kartoffeltransformation mit dem binären Vektorsystem weitaus effizienter als mit intermediären Vektoren funktioniert, wurde dieses chimäre Gen als 3,4 kb langes EcoRI/HindIII-Fragment in die entsprechenden Schnittstellen des binären Vektors BIN19 umkloniert (pS9). Diese Konstruktionen wurden nun sowohl in Tabak Wisconsin 38 (pM14) als auch in die Kartoffelvarietät Berolina (pS9) eingeführt.

Die Analyse der erhaltenen transgenen Tabak- und Kartoffelpflanzen anhand von Northern Blots ergab eine deutliche Aktivität des GUS- Gens in verwundeten Blättern, während in nicht-verwundeten Blättern keine oder nur eine geringe Aktivität des GUS-Gens nachgewiesen wurden konnte. Die Analyse der mRNA und der GUS-Aktivität in Kartoffelknollen ergab interessanterweise eine ca. 10 fach höhere Aktivität des Gens im Vergleich zu der in verwundeten Blättern. Somit beinhaltet der Proteinase-Inhibitor II-Promotor nicht nur die Steuerelemente für die Wundinduktion, sondern auch solche für eine Expression in Kartoffelknollen.


Ausführungsbeispiel 6

Expression von Genen, die für toxische Proteine kodieren, unter der Kontrolle der regulatorischen Bereiche des Proteinase-Inhibitor II-Gens

Eine Anwendung der regulatorischen Bereiche des Proteinase-Inhibitor II-Gens ist die wundspezifische Expression von toxischen Proteinen in Blättern zur Abwehr von Pflanzenschädlingen wie Insekten oder bestimmten Mikroorganismen. Des halb wurde ein Gen, welches für das toxische Thionin kodiert, unter die Kontrolle des Proteinase-Inhibitor II-Promotors gestellt. Thionine werden normalerweise im Endosperm verschiedener Getreide, aber auch in Blättern von Gersten-Keimlingen exprimiert (Bohlmann und Apel, MGG (1987), 207, 446-454). Unter der Kontrolle des Proteinase-Inhibitor II-Promotors konnte dagegen in transgenen Tabakpflanzen eine wundspezifische Akkumulation der Thionin-mRNA in Blättern nachgewiesen werden.


**Ansprüche**

1. Agrobakterien, enthaltend eine DNA-Sequenz einer Expressions-Kassette, auf der die regulatorischen Regionen für die wundinduzierbare transkriptionale Regulation im Stengel und in Blättern sowie für die konstitutive transkriptionale Regulation in der Kartoffelknolle lokalisiert sind.

2. Agrobakterien gemäß Anspruch 1, dadurch gekennzeichnet, daß es sich um Agrobacterium tumefaciens handelt.

3. Agrobacterium tumefaciens gemäß Anspruch 2, dadurch gekennzeichnet, daß die DNA-Sequenz der Expressions-Kassette aus dem 3,4 kb langen EcoRI/HindIII-Fragment besteht.

4. Agrobacterium tumefaciens gemäß Anspruch 3, dadurch gekennzeichnet, daß in der DNA-Sequenz der Expressions-Kassette die Sequenz eines Proteinase-Inhibitor II-Gens enthalten ist.

5. Agrobacterium tumefaciens gemäß Anspruch 4, dadurch gekennzeichnet, daß das Proteinase-Inhibitor II-Gen aus Solanum tuberosum ist.

6. Agrobacterium tumefaciens gemäß Anspruch 3, dadurch gekennzeichnet, daß in der DNA-Sequenz das 0,26 kb lange SphI-SphI-Fragment der proteinase-Inhibitor II-Region enthalten ist.

7. Agrobacterium tumefaciens gemäß Anspruch 3, dadurch gekennzeichnet, daß in der DNA-Sequenz ein Proteinase-Inhibitor II-Promotor enthalten ist.

8. Proteinase-Inhibitor II-Promotor gemäß Anspruch 7, dadurch gekennzeichnet, daß er aus dem 1,3 kb langen Schal/HindIII-Fragment besteht.

9. Vektor pM 14, bestehend aus einer 8,1 kb langen DNA-Sequenz.

10. Vektor pM 14 gemäß Anspruch 9, dadurch gekennzeichnet, daß er das 3,4 kb lange EcoRI/HindIII-DNA-Fragment enthält.

11. Vektor pM gemäß Anspruch 10, dadurch gekennzeichnet, daß in dem DNA-Fragment das 1,3 kb lange Scal/HindIII-Fragment der Promotorregion, das 1,8 kb lange BamHI/SstI-Fragment des ß-Glucuronidase Gens und das 0,26 kb lange SphI/SphI-Fragment der Proteinase-Inhibitor II-Region enthalten ist.

12. Vektor pM 14 gemäß Anspruch 11, dadurch gekennzeichnet, daß das ß-Glucuronidase-Gen 3' hinter dem Proteinase-Inhibitor II-Promotor und 5' vor dem 3'-Ende des Protinase-Inhibitor II-Gens lokalisiert ist.

13. Vektor pM 14 gemäß Anspruch 9, dadurch gekennzeichnet, daß er in Agrobacterium tumefaciens enthalten ist.

14 Ein pflanzliches Genom, dadurch gekennzeichnet, daß es sich um das Genom der Kartoffel oder des Tabaks handelt.

15. Kartoffel gemäß Anspruch 14, dadurch gekennzeichnet, daß sie eine DNA-Sequenz einer Expressions-Kassette, auf der die regulatorischen Regionen für die Wundinduzierbare transkriptionale Regulation im Stengel und in Blättern sowie für die konstitutive transkriptionale Regulation in der Knolle lokalisiert sind, enthält.

16. Kartoffel gemäß Anspruch 15, dadurch gekennzeichnet, daß in der DNA-Sequenz der Expressions-Kassette die Sequenz eines Proteinase-Inhibitor II-Gens enthalten ist.

17. Kartoffel gemäß Anspruch 15, dadurch gekennzeichnet, daß in der DNA-sequenz das 0,26 kb lange SphI/SphI-Fragment der Proteinase-Inhibitor II-Region enthalten ist.

18. Kartoffel gemäß Anspruch 15, dadurch gekennzeichnet, daß in der DNA-Sequenz ein Proteinase-Inhibitor II-Promotor enthalten ist.

19. Proteinase-Inhibitor II-promotor, gemäß Anspruch 18, dadurch gekennzeichnet, daß er aus dem 1,3 kb langen Scal/HindIII-Fragment besteht.

20. Verwendung des Proteinase-Inhibitor II-Gens für die wundinduzierbare transkriptionale Regulation im Stengel und in Blättern sowie für die konstitutive transkriptionle Regulation in Knollen von Kulturpflanzen.

21. Verwendung des Proteinase-Inhibitor II-Gens gemäß Anspruch 20, dadurch gekennzeichnet, daß die Kulturpflanze eine Tabakpflanze ist.

22. Verwendung des Proteinase-Inhibitor II-Gens gemäß Anspruch 20, dadurch gekennzeichnet, daß die Kulturpflanze eine Kartoffelpflanze ist.

23. Verwendung des Proteinase-Inhibitor II-Gens für die wundinduzierbare Expression von Gegen, die für toxische proteine in Blättern, Stengeln und Knollen von Kulturpflanzen kodieren.

24. Verwendung des Proteinase-Inhibitor II-Gens gemäß Anspruch 23, dadurch gekennzeichnet, daß die Kulturpflanze eine Tabakpflanze ist.

25. Verwendung des Proteinase-Inhibitor II-Gens gemäß Anspruch 23, dadurch gekennzeichnet, daß die Kulturpflanze eine Kartoffelpflanze ist.

26. Verwendung des Proteinase-Inhibitor II-Gens gemäß Anspruch 23, dadurch gekennzeichnet, daß das exprimierende Gen für das toxische Protein Thionin kodiert.

27. Verwendung des Proteinase-Inhibitor II-Gens gemäß Anspruch 23, dadurch gekennzeichnet, daß die toxischen Proteine zur Abwehr von Pflanzenschädlingen dienen.

28. Verwendung des Proteinase-Inhibitor II-Gens gemäß Anspruch 27, dadurch gekennzeichnet, daß die toxischen Proteine zur Abwehr von Insekten und Mikroorganismen dienen.

29. Agrobacterium tumefaciens A.tum. M 14 (DSM 5088).

SstI/PstI
SphI
Proteinase-Inhibitorll
(3'-Ende) 0,26 kb
SphI
HindIII

1,8 kb

ß-GLUCURONIDASE

SmaI
ScaI/SmaI
BamHI

pMK1 4
(8,1 kb)

Streptomycin/Spectinomycin

4,74 kb

(Transkriptionsstart)

TATAA

CAAT

23
22
75 bp

PROTEINASE-INHIBITOR II-PROMOTOR

1,3 kb

SmaI/HindIII

EcoRI

☒ = Polylinker-Sequenzen von pMPK 110

☐ = pMPK 110-Sequenzen

Abb. 1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | THE EMBO JOURNAL, Band 6, Nr. 2, Februar 1987, Seiten 303-306, Eynsham, Oxford, GB; J.J. SANCHEZ-SERRANO et al.: "Wound-induced expression of a potato proteinase inhibitor II gene in transgenic tobacco plants" * Das ganze Dokument insbesonders, Seite 306, linke Spalte, Absätze 3,4 * | 1-7,20, 21,23- 28 | C 12 N 1/21 C 12 N 15/82 C 12 N 15/70 A 01 H 5/00 A 01 N 65/00 |
| X | PROC. NATL. ACAD. SCI., (USA) Band 84, Seiten 744-748, 1987 * Das ganze Dokument * | 1,2,20, 21 | |
| A | IDEM | 3-19,22 -29 | |
| P,X | EMBO JOURNAL, Bad 8, Nr. 5, Seiten 1323-1330, 1989; M. KEIL et al.: "Both wound-inducible and tuber-specific expression are mediated by the promoter of a single member of the potato protienase inhibitor II gene family" * Das ganze Dokument * | 1-29 | |
| A | NUCLEIC ACIDS RESEARCH, Band 14, Nr. 14, 1986, Seiten 5641-5650; M. KEIL et al.: "Primary structure of a proteinase inhibitor II gene from potato (Solanum tuberosum)" * Das ganze Dokument * | 1-29 | **RECHERCHIERTE SACHGEBIETE (Int. Cl.5)** C 12 N A 01 H A 01 N |
| A | SCIENCE, Band 237, Seiten 1176-1183; J.St. SCHELL: "Transgenic plants as tools to study the molecular organization of plant genes" * Seite 1180, linke Spalte, Absätze 2,3 * | 1-29 | |

-/-

Der vorliegende Recherchenbericht wurde für alle Patentansprüche erstellt

| Recherchenort | Abschlußdatum der Recherche | Prüfer |
|---|---|---|
| DEN HAAG | 13-02-1990 | MADDOX A.D. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

&: Mitglied der gleichen Patentfamilie, übereinstimmendes Dokument

EPO FORM 1503 03.82 (P0403)

Europäisches
Patentamt

EUROPÄISCHER RECHERCHENBERICHT

Nummer der Anmeldung

EP 89 25 0116

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| O,X | UCLA SYMPSIA. MOL. CELL. BIOL., New SER. 1987, vol. 62, (CIBA-GEIGY UCLA SYMPOSIUM TAMARRON CO. USA), 2nd-8th Februäry 1987, pages 331-338; J. SANCHEZ-SERRANO et al.: "Wound-induced expression of proteinase inhibitor II in potato and transgenic tobacco plants" * Seiten 335-336 * ----- | 1-7,20, 21,23, 24,27, 28 | |

RECHERCHIERTE SACHGEBIETE (Int. Cl.5)

Der vorliegende Recherchenbericht wurde für alle Patentansprüche erstellt

| Recherchenort | Abschlußdatum der Recherche | Prüfer |
|---|---|---|
| DEN HAAG | 13-02-1990 | MADDOX A.D. |

KATEGORIE DER GENANNTEN DOKUMENTE

X : von besonderer Bedeutung allein betrachtet
Y : von besonderer Bedeutung in Verbindung mit einer
   anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
   nach dem Anmeldedatum veröffentlicht worden ist
D : in der Anmeldung angeführtes Dokument
L : aus andern Gründen angeführtes Dokument
........................................................................
& : Mitglied der gleichen Patentfamilie, übereinstimmendes
   Dokument

EPO FORM 1503 03.82 (P0403)